# EUROPEAN PATENT SPECIFICATION

(11) **EP 3 567 563 B1**
(45) Date of publication and mention of the grant of the patent: **13.12.2023**
(21) Application number: 19173609.9
(22) Date of filing: 09.05.2019
(51) Int. Cl.: G08B 21/22, G08B 21/04, A61B 5/11, A61B 5/00

(54) **SYSTEM AND METHOD FOR MONITORING PATIENTS WITH COGNITIVE DISORDERS**
SYSTEM UND VERFAHREN ZUR ÜBERWACHUNG VON PATIENTEN MIT KOGNITIVEN STÖRUNGEN
SYSTÈME ET PROCÉDÉ DE SURVEILLANCE DE PATIENTS SOUFFRANT DE TROUBLES COGNITIFS

(30) Priority: 11.05.2018 IT 201800005262
(43) Date of publication of application: 13.11.2019
(73) Proprietor: Innovazione Tecnologica Srl, 88100 Catanzaro (CZ) (IT); Ricci, Luciano, 88100 Catanzaro (CZ) (IT)
(72) Inventor: RICCI, LUCIANO, 88100 CATANZARO (CZ) (IT)
(74) Representative: Giuliano, Natalia

(56) References cited:
- US-A1- 2003 236 474
- US-A1- 2006 017 558
- US-A1- 2016 140 827
- US-A1- 2017 193 180

## Description

The present invention relates to a system for monitoring patients with cognitive disorders.

The present invention also relates to a method for monitoring patients with cognitive disorders.

In particular, the present invention relates to a system and a method for monitoring patients with cognitive disorders subject to a risk of injuries due to falls and/or loss of consciousness and/or confusion.

In particular, the present invention relates to a system and a method for monitoring patients with cognitive disorders, of the type suffering from a disease such as Alzheimer.

It is known how serious the issues caused by Alzheimer, a disorder which is the most common cause of dementia, are. In fact, Alzheimer constitutes 60% of all cases of dementia and creates, as well as other degenerative neurological disorders, a state of total dependency of the patient, so creating a really complicated and difficult situation for the family who has to assist and also for the health facility that has the task to assist and cure the patient. The assistance weighs for 80% of all cases on the family who has to commit the entire day to cure the patient, with a great psychological stress and expenditures. By the way, even when the treatment of the patient is entrusted to health facilities, these ones suffer from increasing expenditures due to the continuous monitoring that such a disease involves. It is necessary, in fact, to set aside resources and healthcare professionals day and night, with inevitable implications, both technical and economical.

A first example of known system is described in the patent application US2003236474A1 whose subject are an apparatus and a method for monitoring the movements of a patient. If movements of the patient are detected and exceed predetermined thresholds, a detector assembly determines that a seizure condition is present, and an alarm may be generated. In one arrangement, a sensor assembly includes one or more sound detectors to detect sound caused by a patient's movement. The detector assembly monitors the movement of the patient for determining if an abnormal movement or a seizure condition is present. In another arrangement, an image sensor is used. A detector assembly monitors images produced by the image sensor to determine if the movements exceed predetermined thresholds. Other types of sensors can be used in other arrangements. The sensors can either be placed on a surface common to the patient or they can be attached to the patient's body.

Still, the patent application US2016140827A1 describes a person support apparatus, such as a bed, a stretcher, a cot, or the like, and includes a fall detector. The fall detector is adapted to detect when a person associated with the person's support apparatus has fallen and to issue a fall alarm. In some embodiments, the person support apparatus also includes an exit detection system that issues an exit alarm when the person exits from the person support apparatus. The fall alarm is given a higher priority than the exit alarm. The fall detector may include a camera, a thermal image sensor, a device worn by the person, or another sensor. The person support apparatus may also, or alternatively, include a timer for measuring how long an occupant remains out of the person support apparatus.

A further solution published in the patent application US2016/0063846 describes a system for determining the position of a patient in a hospital bed comprising: at least a deformation sensor, able to generate a signal indicating a deformation of the structure of the bed; a unit able to determine the position, in order to determine a lateral and/or longitudinal position of the patient depending on the deformation of the structure. The application also describes a method for monitoring the exit of a patient from a hospital bed comprising the steps of: determining the position of the patient on the bed depending on the measured deformation and generating a warning signal if the determined position is out of a preset area. A system for detecting the weight of a hospital bed comprises a base with a frame suspended with respect to a fixed frame comprising: a load sensor which connects the suspended frame and the fixed frame by means of a suspension element which allows a free vertical movement of the suspended frame with respect to the fixed frame.

Finally, the international application published as WO2014165798 describes a method and an apparatus for detecting and alerting about a patient leaving the bed, this being a factor which causes stress to those who take care of patients who often wander out of bed due to dementia, Alzheimer's disease or other medical conditions. According to an aspect, the apparatus comprises a sock provided with a pressure sensor and a battery-powered microcontroller unit provided with a radiofrequency module. Once the patient leaves the bed and touches the floor, the sensor immediately detects the pressure caused by his body weight and activates by a wireless connection a sound audible at a monitoring unit which can be a smartphone, a tablet or a PC. According to an aspect, the sensor and the microcontroller unit can be assembled in a single assembly to be mounted on a normal sock, slipper or shoe. The device also counts the steps and measures the time interval between them accurately. According to an aspect, the pressure sensor and/or the sensor/microcontroller assembly can adhere to a foot.

However, the known systems are complex and expensive.

The purpose of the present invention is to provide a system and a method for monitoring patients with cognitive disorders that is able to control and monitor the patient in real-time in a reliable, fast and efficient manner, so optimizing the human resources acting both in a clinic and in a family, therefore having characteristics so as to overcome the limits that still affect the actual known systems.

According to the present invention a system for monitoring patients with cognitive disorders is provided, as defined in claim 1.

According to the present invention a method for monitoring patients with cognitive disorders is also provided, as defined in claim 7.

For a better understanding of the present invention, it is now described a preferred embodiment, purely by way of non-limiting example, with reference to the accompanying drawing, in which:

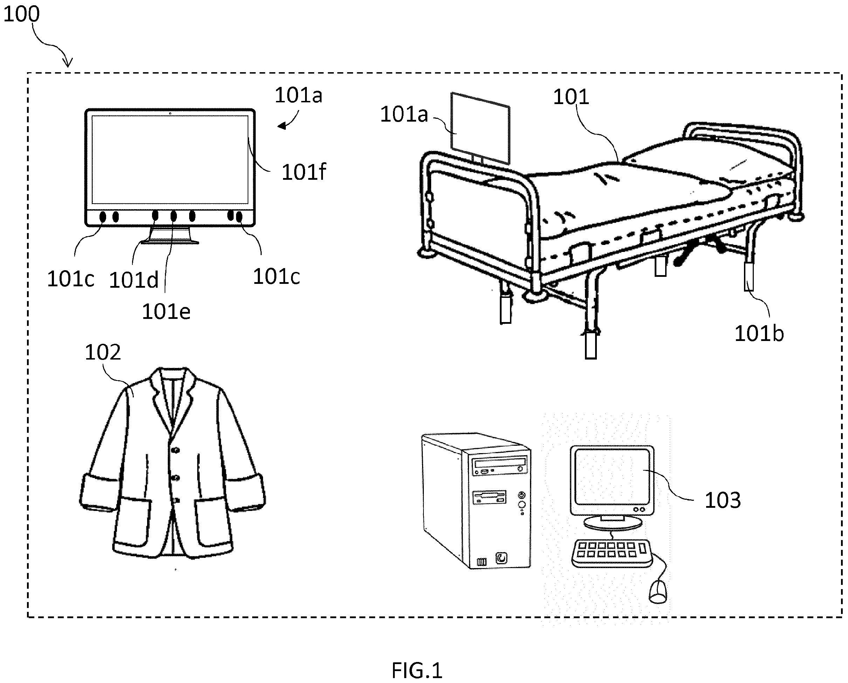

- figure 1 shows a schematic view of a system for monitoring patients with cognitive disorders, according to the invention.

With reference to figure 1, a system 100 for monitoring patients with cognitive disorders is shown, according to the invention.

In particular, the system 100 for monitoring patients with cognitive disorders comprises:
- at least one bed 101 comprising a plurality of weight sensors 101b configured to detect the presence of a patient in the bed 101;
- a plurality of sensors 101c configured to detect the descending of the patient from the bed 101;
- a plurality of acoustic sensors 101e able to detect environmental noises near the bed 101 of the patient;
- a plurality of flame sensors 101d able to detect smoke and flames near the bed 101 of the patient;
- a plurality of shock sensors 101f able to detect sudden movements of the bed 101;
- at least a wearable sensor 102 placed on the garment of the patient;
- at least a processing unit 101a provided with an Internet access and with a control system configured to acquire, digitize and analyze the signals coming from the sensors 101b, 101c, 101e, 101d, 101f, 102 through a machine learning algorithm and to generate visual or acoustic alarms, said visual and acoustic alarms being remotely notified to healthcare professionals provided with mobile devices;
- a video camera mounted on the processing unit 101a;
- a computer 103 provided with an Internet access and configured to receive the data transmitted by the at least a processing unit 101a through a wired or a wireless local network.

According to an aspect of the invention, the weight sensors 101b configured to detect the presence of the patient in the bed 101 are weight sensors of the oleo dynamic type mounted into the feet of the bed 101 and are configured to measure the weight of the bed 101.

Advantageously according to the invention, the use of weight sensors 101b of the oleo dynamic type allows a more accurate measure of the weight of the bed 101 in the presence and in the absence of the patient, compared to weight sensors realized through other technologies.

Another advantage allowed by the use of the weight sensors 101b of the oleo dynamic type according to the invention is that they can be used in any hospital or domestic bed.

Advantageously according to the invention, the acoustic sensors 101e allow to detect environmental noises, for example screams emitted by the patient, whose sound intensity is higher than a preset threshold.

According to the invention, the processing unit 101a is mounted at the foot of the bed.

According to the invention, the sensors 101c, 101e, 101d, 101f are housed into the processing unit 101a.

Advantageously according to the invention, the use of flame sensors 101d allows to detect promptly the presence of fume or flames near the bed 101 of the patient.

According to an aspect of the invention, the control system implemented by the at least a processing unit 101a is based on a machine learning algorithm developed on the basis of the behavioral analysis of the sampled and analyzed data and is able to process through a comparison with known models the signals generated by the sensors 101b, 101c, 101e, 101d, 101f and 102, generating visual and acoustic alarms.

Advantageously according to the invention, said machine learning algorithm allows to obtain a synergic effect on the detections carried out by all the sensors, discriminating and correlating them in a smart way, so minimizing the risk of generating false alarms and notifying the concerning warnings.

The sensor group, in particular, composed as illustrated above, bases its behavior on the analysis of events and is designed depending on said logic of machine learning aiming at discriminating false positives.

The machine learning algorithm that supervises the behavior of the system in its unsupervised learning, used to find a set of frequent behaviors in a given set of data, is based on the "Apriori Machine Learning Algorithm, that is a simple and powerful data mining logic which generates an association rule on the transaction databases and on which some measures have been implemented in order to improve its weak points. The extraction of association rules uses the Candidate-Generation-Based (CGB) and Pattern-Growth-Based (PGB) strategies.

In particular, speaking of sensors having an output with sudden TTL fronts such as the shock sensors 101f (tilt), the algorithm calculates the number of pulses recorded in a given time span obtained from a database of events recorded during the self-learning phase in order to determine whether the detected and processed event from the at least one processing unit 101a is a valid positive or a false positive.

Similarly, although trough different logics, the behavior of the sensors 101c (implemented, for example, through a sonar) is analyzed with regard to detection times and to the speed of the movements through the comparative analysis of predictive models developed experimentally, also crossing data coming from the weight sensors 101b of the oleo dynamic type; the algorithm is however based on a logic capable of self-adaptation in cases where too many false positives are detected.

With reference to the sensors having a linear analogue output such as the flame sensors 101d, the acoustic sensors 101e and the weight sensors 101b, dedicated functions that digitize their measures are carried out through the ADC (Analog to Digital Converter) of the MCU (MicroController Unit) integrated in the at least a processing unit 101a, at regular intervals and in very short times. If in the average of these measurements the potential alarm signal is present only once for a very short period of time <T (T being preset in advance), the algorithm discards the event. On the contrary, if the alarm event is present in more than one measurement made in a time not longer than 2T, the same alarm is notified.

Under conditions of high noises, shocks, vibrations, the functions set themselves up to a predetermined value, in order to try to discriminate the false alarm while remaining within unavoidable and constant limits below or above which the algorithm would not work correctly.

According to another aspect of the invention, each processing unit 101a is associated with a unique identification code which allows the association of the bed and the hospital room with the patient's registry.

According to one aspect of the invention, the processing unit 101a is equipped with a display able to visualize images or videos.

Moreover, according to another aspect of the invention, the at least a processing unit 101a allows to make and receive phone calls and video calls, even without the intervention of the patient, by means of a self-call or an auto-reply.

Advantageously according to the invention, the display allows the patient to visualize images or videos that help him to maintain a connection with his personal memories.

According to another aspect of the invention, the processing unit 101a sends the data stored therein according to a preset programming to a remote cloud server, not shown in the figure.

Advantageously according to the invention, this periodic data upload operation allows both to store data of any single patient, and to verify the correspondence of said data with those stored locally in the processing unit 101a, thus detecting possible manipulations of the same data, voluntary or unintentional, made by patients.

In detail, the architecture of the software modules has been designed according to a criterion of robustness and reliability by historicizing each event detected by a "Domino" central on two redundant servers (a local server and a cloud server) where the access logs of the authorized operators are recorded in order to be able to verify the events detected and their management without the possibility of tampering.

According to another aspect of the invention, and in particular for walking patients, the wearable sensor 102 is coupled with a small proximity sensor, not shown in the figure, that, located in the patient's room, allows to verify the presence of the same patient in the room within the operating range of the sensor 102, which can be set as desired, for example from 0 to 15 m.

Advantageously according to the invention, the voluntary or unintentional departure of the patient from the room can be controlled through the detections of the wearable sensor 102.

According to one aspect of the invention, said proximity sensor is a miniaturized beacon-type device communicating with the wearable sensor 102 through a short-range wireless connection, for example a Bluetooth LE connection.

Furthermore, according to another aspect of the invention, the wearable sensor 102 is configured to detect and notify, through the at least a processing unit 101a, sudden accelerations to which the patient is subject by issuing a distress call in case of an accidental fall or in case of fainting.

According to another aspect of the invention, the system 100 also includes a software application provided with a web user interface that allows to display from afar by the Internet a historical record of the visual and acoustic alarms generated by the processing unit 101a depending on the signals coming from the sensors 101b, 101c, 101d, 101e, 101f and 102, said alarms being sent according to the logic set up by the control system of the processing unit 101a by SMS, instant messaging or e-mail to portable devices, such as smartphones or tablets, of healthcare professionals.

According to another aspect of the invention, said software application provided with a web user interface is installed on the computer 103.

According to another aspect of the invention, the at least a processing unit 101a and the computer 103 are connected through a wired or a wireless LAN network.

As already said, the present invention relates also to a method for monitoring patients with cognitive disorders by means of the system 100, comprising the steps of:
- Detecting the presence or the absence of the patient in a bed through weight sensors of the oleo dynamic type, sudden movements of the bed, presence of flames and smoke near the bed, an attempt to get off the bed made by the patient, the presence of the patient within a preset range of distances;
- Sending the signals of the aforementioned detecting step to a processing unit mounted at the foot of the bed;
- Providing a connection between the processing unit and a device having a software application provided with a web user interface, said device being configured to show a historical record of visual and acoustic warnings generated by the elaboration unit in the aforementioned detecting step.

According to one aspect of the invention, each alarm can be notified to a mobile device of a remote user, for example a healthcare professional provided with a smartphone or a tablet, via SMS, instant messaging or e-mail.

According to another aspect of the invention, the method includes the step of video calling patients.

Advantageously according to the invention, by using the described system 100 and method, a healthcare professional can receive the alarm from one or more patients, going with certainty into the patient's room from which the alarm was generated and thus carry out the due control. Alternatively, the healthcare professional can make a call to the processing unit 101a to monitor the patient's condition from afar.

In addition, advantageously according to the invention, the healthcare professional can:
- manage the alarm by reporting and recording its date, time and type;
- reset any possible malfunctioning or setting up one or more peripherals that may have caused false alarms by changing their thresholds;
- generate a historical record of the alarms received by filtering them by patient's name.

Therefore, the system and method for monitoring patients with cognitive disorders allow a remote monitoring of patients.

Furthermore, the system and method for monitoring patients with cognitive disorders are efficient.

Finally, the system and method for monitoring patients with cognitive disorders allow a real-time monitoring in a reliable, fast and efficient manner, optimizing the human resources acting both in the clinic and in the family, so reducing the effort of a caregiver.

It is finally clear that the system and method for monitoring patients with cognitive disorders described and illustrated herein can be subject to modifications and variations without departing from the scope of the present invention, as defined in the appended claims.

## Claims

1. System (100) for monitoring patients with cognitive disorders comprising:
- at least one bed (101) comprising a plurality of weight sensors (101b) configured to detect the presence of a patient in the bed (101);
- a plurality of sensors (101c) configured to detect the descending of the patient from the bed (101);
- a plurality of acoustic sensors (101e) able to detect environmental noises near the bed (101) of the patient;
- a plurality of flame sensors (101d) able to detect smoke and flames near the bed (101) of the patient;
- a plurality of shock sensors (101f) able to detect sudden movements of the bed (101);
- at least a wearable sensor (102) placed on the garment of the patient;
**characterized in** comprising:
- at least a processing unit (101a), installed at the foot of the bed (101) and housing said sensors (101c) configured to detect the descending of the patient from the bed (101), said acoustic sensors (101e), said flame sensors (101d) and said shock sensors (101f), provided with an Internet access and with a control system configured to acquire, digitize and analyze the signals coming from said weight sensors (101b),said sensors (101c) configured to detect the descending of the patient from the bed (101), said acoustic sensors (101e), said flame sensors (101d), said shock sensors (101f) and said wearable sensor (102) through a machine learning algorithm configured to compare said signals with known models and data related to said signals with a database of events recorded during a self-learning phase generating visual and acoustic alarms remotely notified to healthcare professionals provided with mobile devices, minimizing the risk of generating false alarms;
- a video camera mounted on the processing unit (101a);
- a computer (103) provided with an Internet access and configured to receive the data transmitted by the at least a processing unit (101a) through a wired or a wireless local network.

2. System (100) according to claim 1, **characterized in that** the weight sensors (101b) are weight sensor of the oleo dynamic type and are mounted into the feet of the bed.

3. System (100) according to claim 1, **characterized in that** the processing unit (101a) is associated with a unique identification code of the bed (101) and of the hospital room of the patient.

4. System (100) according to claim 1, **characterized in that** the processing unit (101a) is equipped with a display able to visualize images and is configured to make and receive telephone calls and video calls.

5. System (100) according to claim 1, **characterized in that** the wearable sensor (102) is coupled with a proximity beacon sensor through a short-range wireless connection and is configured to detect and notify sudden accelerations to which the patient is subject.

6. System (100) according to claim 1, **characterized in** comprising a software application provided with a web interface installed on the computer (103) and configured to show a historical record of visual and acoustic alarms generated by the elaboration unit (101a).

7. Method for monitoring a patient with cognitive disorders by means of the system (100) according to claim 1, comprising the steps of:
- Detecting the presence or the absence of the patient in a bed through weight sensors of the oleo dynamic type, sudden movements of the bed, presence of flames and smoke near the bed, an attempt to get off the bed made by the patient, the presence of the patient within a preset range of distances;
- Sending the signals of the aforementioned detecting step to a processing unit (101a) installed at the of the bed;
- Providing a connection between the processing unit (101a) and a device having a software application provided with a web interface, said device being configured to show a historical record of visual and acoustic warnings generated by the elaboration unit in the aforementioned detecting step.

8. Method according to claim 7, **characterized in that** each alarm is able to be notified to a mobile device of a remote user.

9. Method according to claim 7, **characterized in** comprising the step of making a video call.

## Patentansprüche

1. System (100) zur Überwachung von Patienten mit kognitiven Störungen, umfassend:
- mindestens ein Bett (101), das eine Anzahl von Gewichtssensoren (101b) umfasst, die so konfiguriert sind, dass sie die Anwesenheit eines Patienten in dem Bett (101) erkennen;
- eine Anzahl von Sensoren (101c), die so konfiguriert sind, dass sie das Verlassen des Bettes (101) durch den Patienten erfassen;
- eine Anzahl von akustischen Sensoren (101e), die in der Lage sind, Umgebungsgeräusche in der Nähe des Bettes (101) des Patienten zu erfassen;
- eine Anzahl von Brandsensoren (101d), die in der Lage sind, Rauch und Flammen in der Nähe des Bettes (101) des Patienten zu erfassen;
- eine Anzahl von Schocksensoren (101f), die in der Lage sind, plötzliche Bewegungen des Bettes (101) zu erkennen;
- mindestens einen an der Kleidung des Patienten angebrachten tragbaren Sensor (102);
**dadurch gekennzeichnet, dass** sie Folgendes umfasst:
- mindestens eine Verarbeitungseinheit (101a), die am Fußende des Bettes (101) installiert ist und die Sensoren (101c), die so konfiguriert sind, dass sie das Verlassen des Bettes (101) durch den Patienten detektieren, die akustischen Sensoren (101e) , die Brandsensoren (101d) und die Schocksensoren (101f) beherbergt und mit einem Internetzugang und einem Steuersystem ausgestattet ist, das so konfiguriert ist, dass es die von den Gewichtssensoren (101b), den Sensoren (101c), die so konfiguriert sind, dass sie das Verlassen des Bettes (101) durch den Patienten detektieren, kommenden Signale erfasst, digitalisiert und analysiert, den akustischen Sensoren (101e), den Flammensensoren (101d), den Schocksensoren (101f) und dem tragbaren Sensor (102) durch einen Algorithmus für maschinelles Lernen, der so konfiguriert ist, dass er die Signale mit bekannten Modellen und Daten, die sich auf die Signale beziehen, mit einer Datenbank von Ereignissen, die während einer Selbstlernphase aufgezeichnet wurden, vergleicht, wodurch visuelle und akustische Alarme erzeugt werden, die aus der Ferne an medizinisches Fachpersonal, das mit mobilen Geräten ausgestattet ist, gemeldet werden, wodurch das Risiko von Fehlalarmen minimiert wird;
- eine an der Verarbeitungseinheit (101a) montierte Videokamera;
- einen Computer (103), der mit einem Internetzugang ausgestattet und so konfiguriert ist, dass er die von der mindestens einen Verarbeitungseinheit (101a) über ein kabelgebundenes oder ein drahtloses lokales Netz übertragenen Daten empfängt.

2. System (100) nach Anspruch 1, **dadurch gekennzeichnet, dass** die Gewichtssensoren (101b) Gewichtssensoren des oleo-dynamischen Typs sind und in den Füßen des Bettes montiert sind.

3. System (100) nach Anspruch 1, **dadurch gekennzeichnet, dass** die Verarbeitungseinheit (101a) mit einem eindeutigen Identifikationscode des Bettes (101) und des Krankenhauszimmers des Patienten verbunden ist.

4. System (100) nach Anspruch 1, **dadurch gekennzeichnet, dass** die Verarbeitungseinheit (101a) mit einem Bildschirm ausgestattet ist, der in der Lage ist, Bilder zu visualisieren, und der so konfiguriert ist, dass er Telefonanrufe und Videoanrufe tätigen und empfangen kann.

5. System (100) nach Anspruch 1, **dadurch gekennzeichnet, dass** der tragbare Sensor (102) über eine drahtlose Kurzstreckenverbindung mit einem Näherungssensor gekoppelt ist und so konfiguriert ist, dass er plötzliche Beschleunigungen, denen der Patient ausgesetzt ist, erkennt und meldet.

6. System (100) nach Anspruch 1, **dadurch gekennzeichnet, dass** es eine Softwareanwendung mit einer Internetschnittstelle umfasst, die auf dem Computer (103) installiert und so konfiguriert ist, dass sie eine historische Aufzeichnung der visuellen und akustischen Alarme anzeigt, die von der Verarbeitungseinheit (101a) erzeugt werden.

7. Verfahren zur Überwachung eines Patienten mit kognitiven Störungen mittels des Systems (100) nach Anspruch 1, umfassend die Schritte:
- Erkennung der Anwesenheit oder Abwesenheit des Patienten in einem Bett durch Gewichtssensoren vom Typ Oleo-Dynamik, plötzliche Bewegungen des Bettes, Präsenz von Flammen und Rauch in der Nähe des Bettes, Versuch des Patienten, das Bett zu verlassen, Anwesenheit des Patienten innerhalb eines vorgegebenen Abstandsbereichs;
- Weiterleitung der Signale des vorgenannten Erfassungsschritts an eine am Fußende des Bettes installierte Verarbeitungseinheit (101a);
- Vorhandensein einer Verbindung zwischen der Verarbeitungseinheit (101a) und einem Gerät mit einer Softwareanwendung, die mit einer Internetschnittstelle versehen ist, wobei das Gerät so konfiguriert ist, dass es eine historische Aufzeichnung der visuellen und akustischen Warnungen anzeigt, die von der Verarbeitungseinheit in dem vorgenannten Erfassungsschritt erzeugt wurden.

8. Verfahren nach Anspruch 7, **dadurch gekennzeichnet, dass** jeder Alarm an ein Mobilgerät eines entfernten Benutzers gemeldet werden kann.

9. Verfahren nach Anspruch 7, **dadurch gekennzeichnet, dass** es den Schritt umfasst, einen Videoanruf zu tätigen.

## Revendications

1. Système (100) de surveillance de patients atteints de troubles cognitifs comprenant :
- au moins un lit (101) comprenant une pluralité de capteurs de poids (101b) configurés pour détecter la présence du patient dans le lit (101);
- une pluralité de capteurs (101c) configurés pour détecter la descente du patient du lit(101);
- une pluralité de capteurs acoustiques(101e) capables de détecter les bruits ambiants à proximité du lit (101) du patient;
- une pluralité de capteurs flammes (101d) capables de détecter la fumée et les flammes à proximité du lit (101) du patient;
- un pluralité de capteurs de chocs (101f) capables de détecter des mouvements soudains du lit (101);
- au moins un capteur portable (102) placé sur les vêtements du patient ;
**Caractérisé par le fait qu'**il comprend :
- au moins une unité de traitement (101a) installée au pied du lit (101) qui renferme lesdits capteurs(101c) configurés pour détecter la descente du patient du lit (101), lesdits capteurs acoustiques(101e), lesdits capteurs de flammes(101d)et lesdits capteurs de chocs(101f), fournie avec un accès Internet et avec un système de commande configuré pour recueillir, numériser et analyser les signaux émis desdits capteurs de poids (101b), desdits capteurs(101c) configurés pour détecter la descente du patient du lit (101), desdits capteurs acoustiques(101e), desdits capteurs de flammes(101d), desdits capteurs de chocs(101f) et dudit capteur portable(102) par le biais d'un algorithme d'apprentissage automatique conçu pour comparer lesdits signaux avec des modèles et données connus en lien avec lesdits signaux avec une base de données d'évènements enregistrés durant une phase d'auto-apprentissage générant des alarmes visuelles et acoustiques qui sont notifiées à distance à des professionnels de santé équipés d'appareils mobiles, réduisant le risque de générér de fausses alarmes;
- une caméra vidéo montée sur l'unité de traitement(101a) ;
- un ordinateur (103) avec accès à Internet et configuré pour recevoir des données transmises par au moins une unité de traitement(101a) par le biais d'un réseau local câblé ou sans fil.

2. Système (100) selon la revendication 1, **caractérisé par le fait que** les capteurs de poids(101b) sont des capteurs de poids de type oléodynamique et qu'ils sont montés dans les pieds du lit.

3. Système (100) selon la revendication 1, **caractérisé par le fait que** l'unité de traitement(101a) est associée à un code d'identification unique du lit (101) et de la chambre d'hôpital du patient.

4. Système (100) selon la revendication 1, **caractérisé par le fait que** l'unité de traitement(101a) est équipée d'un écran capable de visualiser des images et est configuré de manière à passer et recevoir des appels téléphoniques et des appels vidéos.

5. Système (100) selon la revendication 1, **caractérisé par le fait que** le capteur portable (102) est couplé à un capteur beacon de proximité par le biais d'une connexion sans fil à courte portée et est configuré pour détecter et notifier les accélérations soudaines auxquelles le patient est soumis.

6. Système (100) selon la revendication 1, **caractérisé en ce qu'**il comprend une application logicielle dotée d'une interface web installée sur l'ordinateur (103) et configurée pour afficher un historique des alarmes visuelles et acoustiques générées par l'unité d'élaboration (101a).

7. Méthode de surveillance d'un patient atteint de troubles cognitifs au moyen du système (100) selon la revendication 1, comprenant les étapes suivantes :
- Détection de la présence ou l'absence du patient du lit par le biais de capteurs de poids de type oléodynamique, de mouvements soudains du lit, de la présence de flammes et de fumée à proximité du lit, d'une tentative du patient de descendre du lit, de la présence du patient dans une zone de distances prédéterminées;
- Envoi des signaux de l'étape de détection susmentionnée à une unité de traitement (101a) installée au pied du lit ;
- Établissement d'une connexion entre l'unité de traitement(101a) et un dispositif doté d'une application logicielle avec une interface web, ledit dispositif étant configuré pour afficher un historique des avertissements visuels et acoustiques générés par l'unité d'élaboration au cours de l'étape de détection susmentionnée.

8. Méthode selon la revendication 7, **caractérisée par le fait que** chaque alarme peut être notifiée à un appareil mobile d'un utilisateur se situant à distance.

9. Méthode selon la revendication 7, **caractérisée par** l'étape consistant à passer un appel vidéo.
